**Europäisches Patentamt**

**European Patent Office**

**Office européen des brevets**

(19)

(11) Veröffentlichungsnummer: **0 259 383**
**B1**

(12) **EUROPÄISCHE PATENTSCHRIFT**

(45) Veröffentlichungstag der Patentschrift: **23.01.91**

(51) Int. Cl.⁵: **A 61 K 9/12, A 61 K 31/55**

(21) Anmeldenummer: **87901316.7**

(22) Anmeldetag: **10.03.87**

(86) Internationale Anmeldenummer:
**PCT/AT87/00015**

(87) Internationale Veröffentlichungsnummer:
**WO 87/05210 11.09.87 Gazette 87/20**

(54) **PHARMAZEUTIKUM SOWIE VERFAHREN ZU SEINER HERSTELLUNG.**

(30) Priorität: **10.03.86 AT 621/86**

(43) Veröffentlichungstag der Anmeldung:
**16.03.88 Patentblatt 88/11**

(45) Bekanntmachung des Hinweises auf die
Patenterteilung:
**23.01.91 Patentblatt 91/04**

(84) Benannte Vertragsstaaten:
**AT BE CH DE FR GB IT LI LU NL SE**

(56) Entgegenhaltungen:
**WO-A-81/03421**
**BE-A- 856 652**

**R. Voigt Lehrbuch der pharmazeutischen
Technologie (1984), Seite 429**

(73) Patentinhaber: **Burghart, Kurt, Dr.
Sägeberg 8
D-2217 Rosdorf (DE)**
(73) Patentinhaber: **Burghart, Walter
Salmgasse 4
A-1030 Wien (AT)**

(72) Erfinder: **Burghart, Kurt, Dr.
Sägeberg 8
D-2217 Rosdorf (DE)**
Erfinder: **Burghart, Walter
Salmgasse 4
A-1030 Wien (AT)**

(74) Vertreter: **Haffner, Thomas M., Dr. et al
Patentanwaltskanzlei Dipl.-Ing. Adolf
Kretschmer Dr. Thomas M. Haffner
Schottengasse 3a
A-1014 Wien (AT)**

**EP 0 259 383 B1**

**Beschreibung**

Die Erfindung bezieht sich auf ein Pharmazeutikum mit Benzodiazepinen als Wirkstoff sowie auf ein Verfahren zu seiner Herstellung.

Benzodiazepine, im besonderen das 1,3 Dihydro-7-Nitro-5-phenyl-2H-1,4-Benzodiazepin-2-on oder das Diazepam (7-Chlor-1,3-dihydro-1-Methyl-5-phenyl-2H-1,4-Benzo-diazepin-2-on oder Fluorazepam (7-Chlor-1-2-(diäthylamino)ethyl-5-(2-fluorphenyl)-1H-1,4-benzodiazepin-2(3H)-on oder Triazolam 8-Chlor-6-(o-Chlorphenyl)-1-methyl-4H-s-triazolo-[4,3-a]-[1,4]benzodiazepin oder Alprazolam 8-Chlor-l-methyl-6-phenyl-4H-1,2,4-triazolo-[4,3-a]-[1,4]benzodiazepin oder Midazolam 8-Chlor-6-(2-fluorphenyl)-1-methyl-4H-imidazolo[1,5-a]-[1,4]benzodiazepin sind als Beruhigungsmittel stark verbreitet und werden, insbesondere bei Schlafstörungen, Angstgefühlen im Status epilepticus, bei Spasmen, zentralnervöser Genese od.dgl. eingesetzt. Die übliche Darreichungsform derartiger Benzodiazepine besteht in Form von Tabletten, wobei der Wirkungseintritt üblicherweise erst nach einiger Zeit zu erwarten ist, wobei besonders nach vorheriger Nahrungsaufnahme eine stark verzögerte Wirkung eintreten kann. Eine Formulierung mit verbesserter Resorbierbarkeit und rascherem Wirkungseintritt wurde in Form einer Tropflösung vorgeschlagen. Derartige Tropflösungen erfordern für das Dosieren eine relativ ruhige Hand und sind, insbesondere beispielsweise bei Anwendungen im Zusammenhang mit Status epilepticus. oder Spasmen zentralnervöser Genese dem Patienten kaum zuzumuten.

In der DE-OS 28 30 044 wurden bereits pharmazeutische Benzodiazepin enthaltende Präperate beschrieben, deren Verabreichung in Form von Tropfen oder Weichgelatine-Kapseln erfolgt.

Aus der BE-PS 856 652 sind pharmazeutische Präparate entnehmbar, welche als Wirkstoff in organischen Lösungsmitteln gelöste Benzodiazepin-Hydrochloride enthalten, und welche in Form von Weichgelatinekapseln oder Tropfen verabreicht werden.

Der genannten BE-PS 856 652 sind Lösungen eines Hydrochlorides eines Benzodiazepines zu entnehmen, welche tatsächlich ohne Treibgas und präzise gesprochen, nur ohne Treibgas als Lösungen angesprochen werden können. Das genannte Hydrochlorid ist nämlich eine polare Substanz, die in Anwesenheit von Treibgas zum Ausfällen tendiert, und eigene Versuche haben ergeben, daß ein derartiges Ausfallen beim Zumischen von Treibgas sofort beobachtet wird. Weiters gilt für die Lösung gemäß Anspruch 1 der genannten belgischen Patentschrift, daß die Lösungsmittel 1,2-Propandiol, 1,3-Propandiol und Glycerin in keiner Weise mit Treibgas mischbar sind und daher Lösungen unter Verwendung dieser Lösungsmittel für die erfindungsgemäßen Zwecke ungeeignet sind.

Mit Rücksicht auf die oben ausgeführten Unterschiede zum Gegenstand der BE-PS 856 652 erscheint der Hinweis auf das Lehrbuch der pharm. Technologie 1984, Seite 429, geeignet, die Erfindungseigenschaft zu unterstreichen. Die bekannten Lösungen waren nämlich offensichtlich nicht geeignet, in einfacher Weise ein Aerosol mit verbesserter Bioverfügbarkeit zu ergeben. Die Aussage in dem genannten Lehrbuch, gemäß welcher in Aerosolform applizierte Arzneimittel "im allgemeinen" außerordentlich schnell resorbiert werden, ist im übrigen zweifelsfrei nicht generell gültig. Für eine Reihe spezieller Wirkstoffe gilt nämlich, daß sie über die Mundschleimhaut nicht in hinreichender Geschwindigkeit resorbiert werden können. Zu derartigen Wirkstoffen zählt beispielsweise Norphenefrin. Von einer Verbesserung der Bioverfügbarkeit kann prinzipiell nur dann gesprochen werden, wenn gleichzeitig eine Verbesserung des first pass-Effektes eintritt, was gleichfalls nicht generell vorausgesetzt werden darf.

Die Erfindung zielt nun darauf ab, derartige bekannte Wirkstoffe in eine Darreichungsform zu bringen, welche eine wesentlich bessere Dosierbarkeit und eine raschere Bioverfügbarkeit und insbesondere von vorheriger Nahrungsaufnahme unabhängige Resorption ergibt und gegenüber der Gefahr der Manipulation des Pharmazeutikums weitgehend geschützt ist. Mit Rücksicht auf die überaus weite Verbreitung derartiger Pharmazeutika kann beispielsweise bei Darreichungsformen in Tropfenform oder auch in Tablettenform leicht der Versuch unternommen werden toxische Substanzen beizumengen. Bei relativ langsamem Wirkungseintritt, wie beispielsweise bei der Verabreichung in Tablettenform ergibt sich darüberhinaus ebenso wie bei unexakter Zählung bei Verabreichung in Tropfenform eine zumeist überflüssig hohe Wirkstoffbelastung, welche durch rascheren Wirkungseintritt vermieden werden könnte. Gerade bei Schlafstörungen und Angstzuständen tendiert der Patient dazu bei ungenügend raschem Wirkungseintritt neuerlich eine zusätzliche Dosis zu sich zu nehmen. Die Erfindung zielt somit darauf ab, die obengenannten Nachteile zu vermeiden und im besonderen eine Formulierung zu schaffen, welche sich durch überaus raschen Wirkungseintritt und überaus geringe Manipulierbarkeit auszeichnet, wobei weiters darauf abgezielt wird die Dosierbarkeit zu verbessern und die Wirkstoffbelastung insgesamt zu verringern. Zur Lösung dieser Aufgabe besteht das erfindungsgemäße Pharmazeutikum mit Benzodiazepinen als Wirkstoff im wesentlichen darin, daß der Wirkstoff in einem Polyalkylenglykol, wie Polyäthylenglykol, und/oder Glycerin-Polyäthylenglykoloxyfettsäureester wie Glycerin-Polyäthylenglykoloxyoleat und/oder Glycerin-Polyäthylenglykoloxystearat, und/oder partiellen Fettsäureestern des Sorbitans bzw. des Polyhydroxyäthylensorbitans, und/oder Polyvinylpyrrolidonen, und/oder Polyvinylalkoholen, und/oder Polyhydroxyäthylenfettalkoholäthern bzw. Polyhydroxyäthylenfettsäureestern und/oder Polyhydroxyäthylen-polyhydroxypropylenkondensaten und/oder Propylencarbonat gelöst mit Äthanol, das gegebenenfalls teilweise durch mittelkettige Fettsäuredi- und/oder -triglyceride ersetzt ist und einem pharmakologisch verträglichen Treibgas, insbesondere halogenierten Kohlenwasserstoffen in Behältnissen als Aerosol dosierbar versprühbar vorliegt. Ein derartiges Aerosol hat überraschenderweise

2

durch die Wahl der erfindungsgemäß vorgeschlagenen Lösungsmittel eine überaus rasche Resorption und einen wesentlich rascheren Wirkungseintritt und eine gleichmäßigere Wirkung als andere Darreichungsformen ergeben. Bedingt durch diesen rascheren Wirkungseintritt besteht eine wesentlich geringere Gefahr, daß der Patient aus Ungeduld zu einer höheren Dosis greift, wodurch die Wirkstoffbelastung wesentlich herabgesetzt wird. Sprühstöße können mit einem Dosierventil exakt dosiert werden und die Dosierbarkeit eines derartigen Aerosols ist der Dosierbarkeit durch Abzählen von Tropfen wesentlich überlegen. Die als Wirkstoff verwendeten Benzodiazepine erfordern in der Regel einen Lichtschutz und die Maßnahme, den Wirkstoff mit den genannten Lösungsmitteln und Äthanol und Treibgas in Dosen abzufüllen, ergibt automatisch den gewünschten Lichtschutz, wenn ein lichtgeschütztes Behältnis gewählt wird. Durch die sublinguale Applikation wird die Mißbrauchsgefahr gleichzeitig erheblich reduziert, da bei Verabreichung vieler Sprühstöße nur mehr oral dar geboten wird und damit eine wesentlich geringere Wirkstoffkonzentration im Blut bzw. physiologische Wirkung eintritt. Weiters ergibt sich bei eingeschränkter Leberfunktion des Patienten der Vorteil, daß sublingual geringere abzubauende Mengen verabreicht werden können.

Besonders bevorzugt ist im Rahmen der Erfindung eine pharmazeutische Zubereitung, welche dadurch gekennzeichnet ist, daß jeder Sprühstoß bezogen auf ein Volumen von 100 µl 0,1 5 mg, Wirkstoff, 3-50 mg, insbesondere 5-25 mg Polyäthylenglykol, und/oder Glycerin-Polyäthylenglykoloxystearat, und oder partielle Fettsäureester des Sorbitans bzw. des Polyhydroxyäthylensorbitans, und/oder Polyvinylpyrrolidone, und/oder Polyvinylalkohole, und/oder polyhydroxyäthylenfettalkoholäther bzw. Polyhydroxyäthylenfettsäureester und/oder Polyhydroxyäthylen-polyhydroxypropylenkondensate, 10-40 mg, insbesondere 15 30 mg, Äthanol, Rest Treibgas und gegebenenfalls pharmazeutische Hilfsstoffe und/oder Aromastoffe enthält. Bei Sprühstößen, deren Volumen 100 µl wesentlich übersteigt, kann die Dosierung an Wirkstoffen je 100 µl nahe der angegebenen Untergrenze gewählt werden.

Besonders bevorzugt ist im Rahmen der vorliegenden Erfindung eine pharmazeutische Zubereitung die dadurch gekennzeichnet ist, daß bei Verwendung von pharmakologisch stark wirksamen Benzodiazepinen, insbesondere von Triazolam als Wirksubstanz die Lösung 0,05-0,3 mg Triazolam je 100 mg Lösung oder je Sprühstoß enthält. Durch die Verwendung von pharmakologisch stark wirksamer Benzodiazepine, insbesondere Triazolam als Wirkstoff kann die Menge an eingesetzter Wirksubstanz etwa um den Faktor 2 gegenüber den anderen bekannten Dosierungen derselben Wirksubstanz als Kapsel oder Tablette und auch als Wirksubstanz einsetzbaren Diazepinen abgesenkt werden.

Vorzugsweise werden bei einer pharmazeutischen Zusammensetzung Benzodiazepine, insbesondere Triazolam und polyäthylenglykol als Lösungsvermittler in einem Verhältnis von 1:20 bis 1:60 vorzugsweise 1:25 bis 1:35 eingesetzt.

Vorzugsweise werden bei einer pharmazeutischen Zusammensetzung Benzodiazepine, insbesondere Diazepam und Propylencarbonat und/oder Glycerin-Polyäthylenglykoloxystearat und/oder mittelkettige Fettsäuredi- und/oder -triglyceride als Lösungsvermittler im Verhältis 1:10 bis 1:20 eingesetzt.

Das erfindungsgemäße Verfahren zur Herstellung eines derartigen Pharmazeutikums mit Benzodiazepinen als Wirkstoff, ist im wesentlichen dadurch gekennzeichnet, daß 0,1-5 Gewichtsteile Diazepine in 3-50 Gewichtsteile eines Lösungsmittels, aus der Gruppe: polyäthylenglykol, und/oder Glycerin-Polyäthylenglykoloxyfettsäure wie Glycerin-Polyäthylenglykoloxyoleat und/oder Glycerin-Polyäthylenglykoloxystearat, und oder partielle Fettsäureester des Sorbitans bzw. des Polyhydroxyäthylensorbitans, und/oder Polyvinylpyrrolidone, und/oder Polyvinylalkohole, und/oder Polyhydroxyäthylenfettalkoholäther bzw. polyhydroxyäthylenfettsäureester und/oder polyhydroxyäthylen-polyhydroxypropylenkondensate und/oder Propylencarbonat gelöst werden und daß die Lösung mit 10 bis 40 Gewichtsteilen Äthanol, das gegebenenfalls teilweise durch mittelkettige Fettsäuredi- und/oder -triglyceride ersetzt ist und 20-70 Gewichtsteilen Treibgas auf der Basis halogenierter Kohlenwasserstoffe in Behältnissen zur Verabreichung als Aerosol mit Sprühstößen zwischen 35 und 500 µl abgefüllt werden.

Bevorzugt wird das erfindungsgemäße Aerosol sublingual angewendet, wobei auf diese Weise der überaus rasche Wirkungseintritt sichergestellt ist.

Die Erfindung wird nachfolgend an Ausführungsbeispielen näher erläutert, wobei als Benzodiazepin 8-Chlor-6-(o-chlorphenyl)-1-methyl-4H-s-triazolo[4,3-a] -[1,4] benzodiazepin (Triazolam) oder 7-Chlor-l,3-dihydro-l-Methyl-5-phenyl-2H-l,4-benzodiazepin-2-on (Diazepam) eingesetzt wurde.

## Beispiel 1

Es wurde eine Lösung bereitet, in welcher 150 mg Aerosol folgende Bestandteile in den angegebenen Mengen enthalten:

| | |
|---|---|
| Diazepam | 2 mg. |
| Polyäthylenglykol 600 | 28 mg. |
| Glycerin-Polyäthylenglykoloxystearat | 28 mg. |
| Äthanol | 30 mg. |
| Treibgas | 90 mg. |
| | 150 mg. |

3

# EP 0 259 383 B1

**Patentansprüche**

1. Pharmazeutikum mit Benzodiazepinen als Wirkstoff, dadurch gekennzeichnet, daß der Wirkstoff in einem Polyalkylenglykol, wie Polyäthylenglykol, und/oder Glycerin-Polyäthylenglykolfettsäureester wie Glycerin-Polyäthylenglykoloxyoleat und/oder Glycerin-polyäthylenglykoloxystearat, und /oder partiellen Fettsäureestern des Sorbitans bzw. des Polyhydroxyäthylensorbitans, und/oder Polyvinylpyrrolidonen und/oder Polyvinylalkoholen, und/oder Polyhydroxyäthylenfettalkoholäthern bzw. Polyhydroxyäthylenfettsäureestern und/oder Polyhydroxyäthylen-polyhydroxypropylenkondensaten und/ oder Propylencarbonat gelöst mit Äthanol, das gegebenenfalls teilweise durch mittelkettige Fettsäuredi- und/oder -triglyceride ersetzt ist und einem pharmakologisch verträglichen Treibgas, insbesondere halogenierten Kohlenwasserstoffen in Behältnissen als Aerosol dosierbar versprühbar vorliegt.

2. Pharmazeutikum nach Anspruch 1, dadurch gekennzeichnet daß jeder Sprühstoß bezogen auf ein Volumen von 100 µl 0,1-5 mg Wirkstoff, 3-50 mg, insbesondere 5-25 mg Polyäthylenglykol, und/oder Glycerin-Polyäthylenglykoloxyfettsäureester wie Glycerin-Polyäthylenglykoloxyoleat und/oder Glycerin-Polyäthylenglykoloxystearat, und/oder partielle Fettsäureester des Sorbitans bzw. des Polyhydroxyäthylensorbitans, und/oder Polyvinylpyrrolidone, und/oder Polyvinylalkohole, und/oder Polyhydroxyäthylenfettalkoholäther bzw. Polyhydroxyäthylenfettsäureester und/oder polyhydroxyäthylen-polyhydroxypropylenkondensate und/oder Propylencarbonat, 10-40 mg, insbesondere 15-30 mg, Äthanol, das gegebenenfalls teilweise durch mittelkettige Fettsäuredi- und/oder -triglycerice ersetzt ist, Rest Treibgas und gegebenenfalls pharmazeutische Hilfsstoffe und/oder Aromastoffe enthält.

3. Pharmazeutikum nach Anspruch 1, dadurch gekennzeichnet, daß bei Verwendung von Triazolam als Wirksubstanz die Lösung 0,05-0,3 mg Triazolam je 100 mg Lösung oder je Sprühstoß enthält.

4. Pharmazeutikum nach Anspruch 3, dadurch gekennzeichnet, daß Benzodiazepine, insbesondere Triazolam und Polyäthylenglykol als Lösungsvermittler in einem Verhältnis von 1:20 bis 1:60 vorzugsweise 1:25 bis 1:35 eingesetzt sind.

5. Pharmazeutikum nach Anspruch 1 oder 2, dadurch gekennzeichnet, daß Benzodiazepine, insbesondere Diazepam und Propylencarbonat und/oder Glycerin-polyäthylenglykoloxystearat und/oder mittelkettige Fettsäuredi- und/oder -triglyceride als Lösungsvermittler im Verhältnis 1:10 bzw. 1:20 eingesetzt sind.

6. Verfahren zur Herstellung eines Pharmazeutikums mit Benzodiazepinen als Wirkstoff dadurch gekennzeichnet, daß 0,1-5 Gewichtsteile Diazepine in 3-50 Gewichtsteile eines Lösungsmittels, aus der Gruppe: Polyalkylenglykol, Polyäthylenglykol, und/oder Glycerin-Polyäthylenglykoloxyfettsäureester wie Glycerin-polyäthylenglykoloxyoleat und/oder Glycerin-Polyäthylenglykoloxystearat, und/oder partielle Fettsäureester des Sorbitans bzw. des Polyhydroxyäthylensorbitans, und/oder Polyvinylpyrrolidone, und/ oder Polyvinylalkohole, und/oder Polyhydroxyäthylenfettalkoholäther bzw. Polyhydroxyäthylenfettsäureester und/oder Polyhydroxyäthylen-polyhydroxypropylenkondensate und/ oder propylencarbonat, gelöst werden und daß die Lösung mit 10 bis 40 Gewichtsteilen Äthanol, das gegebenenfalls teilweise durch mittelkettige Fettsäuredi- und/oder -triglyceride ersetzt ist, und 20-70 Gewichtsteilen Treibgas auf der Basis halogenierter Kohlenwasserstoffe in Behältnissen zur Verabreichung als Aerosol mit Sprühstößen zwischen 35 und 500 µl abgefüllt werden.

**Revendications**

1. Produit pharmaceutique comportant des benzodiazépines comme substance active, caractérisé en ce que la substance active, dissoute dans un polyalkylèneglycol, comme le polyéthylèneglycol, et/ou un ester d'acide gras de glycérol-polyéthylèneglycol comme l'oléate de glycérol-polyéthylèneglycol et/ou le stéarate de glycérol-polyéthylèneglycol et/ou des esters partiels d'acide gras de sorbitanne ou de polyhydroxyéthylènesorbitanne et/ou des polyvinylpyrrolidones et/ou des poly (alcools vinyliques) et/ou des éthers polyhydroxyéthyléniques d'alcools gras ou des esters polyhydroxyéthyléniques d'acides gras et/ou des produits de condensation polyhydroxyéthylène-polyhydroxypropylène et/ou du carbonate de propylène avec de l'éthanol, qui peut éventuellement être partiellement remplacé par des di- et/ou triglycérides d'acides gras à longueur moyenne de chaine, et avec un gaz propulseur pharmacologiquement acceptable, en particulier des hydrocarbures halogénés, est présente dans des récipients, sous forme d'un produit pulvérisable de manière dosable en aérosol.

2. Produit pharmaceutique selon la revendication 1, caractérisé en ce que chaque coup de pulvérisation contient, pour un volume de 100 µl, 0,1 à 5 mg de la substance active, 3 à 50 mg, notamment 5 à 25 mg de polyéthylèneglycol et/ou d'ester d'acide gras de glycérol-polyéthylèneglycol comme de l'oléate de glycérol-polyéthylèneglycol et/ou du stéarate de glycérol-polyéthylèneglycol, et/ou des esters partiels d'acide gras de sorbitanne et/ou de polyhydroxyéthylène sorbitanne et/ou de polyvinylpyrrolidones et/ou de poly(alcools vinyliques) et/ou d'éthers polyhydroxyéthyléniques d'alcools gras ou d'esters polyhydroxyéthyléniques d'acides gras et/ou de produits de condensation polyhydroxyéthylène-polyhydroxypropylène et/ou de carbonate de propylène, 10 à 40 mg, notamment 15 à 30 mg d'éthanol, qui peut éventuellement être partiellement remplacé par des di- et/ou des triglycérides d'acides gras à longueur moyenne de chaîne, le reste étant du gaz propulseur et éventuellement des adjuvants pharmaceutiques et/ou des substances d'aromatisation.

4

# EP 0 259 383 B1

3. Produit pharmaceutique selon la revendication 1, caractérisé en ce qu'en cas d'utilisation de triazolam comme substance active, la solution contient 0,05 à 3 mg de triazolam pour 100 mg de solution ou par coup de pulvérisation.

4. Produit pharmaceutique selon la revendication 3, caractérisé en ce que les benzodiazépines, notamment le triazolam, et le polyéthylène glycol comme tiers solvant sont utilisés selon un rapport de 1:20 à 1:60, avantageusement 1:25 à 1:35.

5. Produit pharmaceutique selon la revendication 1 ou 2, caractérisé en ce que les benzodiazépines, en particulier le diazepam, et le carbonate de propylène et/ou le stéarate de glycérol-polyéthylèneglycol et/ou les di- et/ou tri-glycérides d'acides gras à longueur moyenne de chaîne comme tiers solvant, sont utilisés selon un rapport de 1:10 ou de 1:20.

6. Procédé de préparation d'un produit pharmaceutique contenant des benzodiazépines comme substance active, caractérisé en ce qu'on dissout 0,1-5 parties en poids de diazépines dans 3 à 50 parties en poids d'un solvant du groupe: polyalkylèneglycol, polyéthylèneglycol et/ou ester d'acide gras de glycérol-poléthylèneglycol comme l'oléate de glycérol-polyéthylèneglycol et/ou le stéarate de glycérol-polyéthyléneglycol, et/ou des esters partiels d'acides gras du sorbitanne ou du polyhydroxyéthyléne sorbitanne, et/ou des polyvinylpyrrolidones et/ou des poly (alcools viny liques), et/ou des éthers polyhydroxyéthyléniques d'alcools gras ou des esters polyhydroxyéthyléniques d'acides gras et/ou des produits de condensation polyhydroxyéthylène-polyhydroxypropylène, et l'on introduit la solution, avec 10 à 40 parties en poids d'éthanol, qui peut éventuellement être partiellement remplacé par des di- et/ou triglycérides d'acides gras à longueur moyenne de chaîne, et 20 à 70 parties en poids de gaz propulseur à base d'hydrocarbures halogénés, dans des récipients pour administration sous forme d'aérosol avec des coups de pulvérisation (d'un volume) compris entre 35 et 500 µl.

## Claims

1. A pharmaceutical composition with benzodiazepines as active substance, characterised in that the active substance is presented in metred-dose aerosol containers in a polyalkyleneglycol such as polyethyleneglycol; and/or glyceryl polyethyleneglycol fatty acid esters such as glyceryl polyethyleneglycoloxyoleate and/or glyceryl polyethyleneglycoloxystearate; and/or partial fatty acid esters of sorbitol or polyhydroxyethylene sorbitol; and/or polyvinyl pyrrolidones and/or polyvinyl alcohols; and/or polyhydroxyethylene fatty alcohol ethers or polyhydroxyethylene fatty acid esters; and/or polyhydroxyethylene-polyhydroxypropylene condensates; and/or propylene carbonate dissolved with ethanol which, where appropriate, is partially replaced by medium-chain fatty acid diglycerides and/or triglycerides; and a pharmacologically compatible propellant gas, in particular halogenated hydrocarbons.

2. A pharmaceutical composition according to Claim 1, characterised in that each dose of spray, related to a volume of 100 µl contains 0.1—5 mg of active substance; 3—50 mg, in particular 5—25 mg of polyethyleneglycol; and/or glyceryl polyethyleneglycoloxy fatty acid esters such as glyceryl polyethyleneglycoloxyoleate and/or glyceryl polyethyleneglycoloxystearate; and/or partial fatty acid esters of sorbitol or of polyhydroxyethylene sorbitol; and/or polyvinyl pyrrolidones, and/or polyvinyl alcohols; and/or polyhydroxyethylene fatty alcohol ethers or polyhydroxyethylene fatty acid esters; and/or polyhydroxyethylene-polyhydroxypropylene condensates; and/or propylene carbonate; 10—40 mg, in particular 15—30 mg, ethanol which, where appropriate, is partially replaced by medium-chain fatty acid diglycerides and/or triglycerides; the remainder being propellant gas and, where appropriate, pharmaceutical excipients and/or aromatic substances.

3. A pharmaceutical composition according to Claim 1, characterised in that, when triazolam is used as active substance, the solution contains 0.05—0.3 mg triazolam per 100 mg solution or per dose of spray.

4. A pharmaceutical composition according to Claim 3, characterised in that benzodiazepines, in particular triazolam, and polyethyleneglycol, as solution mediator, are used in a ratio of 1:20 to 1:60, preferably 1:25 to 1:35.

5. A pharmaceutical composition according to Claim 1 or 2, characterised in that benzodiazepines, in particular diazepam, and propylene carbonate and/or glyceryl polyethyleneglycoloxystearate and/or medium-chain fatty acid diglycerides and/or triglycerides as solution mediator are used in the ratio 1:10 and/or 1:20.

6. A method of producing a pharmaceutical composition with benzodiazepines as active substance, characterised in that 0.1—5 parts by weight of diazepines are dissolved in 3—50 parts by weight of a solvent from the group: polyalkyleneglycol; polyethyleneglycol; and/or glyceryl polyethyleneglycoloxy fatty acid esters such as glyceryl polyethyleneglycoloxyoleate and/or glyceryl polyethyleneglycoloxystearate; and/or partial fatty acid esters of sorbitol and/or of polyhydroxyethylene sorbitol; and/or polyvinyl pyrrolidones, and/or polyvinyl alcohols; and/or polyhydroxyethylene fatty alcohol ethers or polyhydroxyethylene fatty acid esters; and/or polyhydroxyethylene-polyhydroxypropylene condensates; and/or propylene carbonate; and in that the solution, together with 10 to 40 parts by weight of ethanol which, where appropriate, is partially replaced by medium-chain fatty-acid diglycerides and/or triglycerides, and 20—70 parts by weight of propellant gas based on halogenated hydrocarbons, is filled into containers for delivery as an aerosol with doses of spray of between 35 and 500 µl.